# FASCICULE DE BREVET EUROPEEN

(11) **EP 1 574 208 B1**
(45) Date de publication et mention de la délivrance du brevet: **10.08.2016**
(21) Numéro de dépôt: 05290322.6
(22) Date de dépôt: 14.02.2005
(51) Int. Cl.: A61K 8/02, A61Q 1/14, A61Q 19/00, A61Q 19/04, A61Q 19/10, D04H 1/425, D04H 1/4258, D04H 1/4291, D04H 1/4334, D04H 3/011, D04H 3/11, D04H 13/00, D04H 1/492, D04H 1/498, D04H 1/435, D04H 3/007

(54) **Article cosmétique à usage unique**
Kosmetische Artikel zum einmaligen Gebrauch
Disposable cosmetic article

(30) Priorité: 08.03.2004 FR 0450469
(43) Date de publication de la demande: 14.09.2005
(73) Titulaire: L'Oréal, 75008 Paris (FR)
(72) Inventeur: Simon, Pascal, 94320 Thiais (FR); Charbossant-Roche, Anne-Clotilde, 75012 Paris (FR)
(74) Mandataire: Nony

(56) Documents cités:
- EP-A- 0 750 062
- EP-A- 1 264 561
- EP-A- 1 352 950
- WO-A-99/25318
- WO-A-03/043551
- US-A- 4 948 585
- US-A- 5 620 694
- US-A- 6 042 844
- US-A1- 2002 155 772

## Description

La présente invention a trait à un article à usage unique imprégné d'une composition cosmétique, notamment pour la peau, les muqueuses, les cheveux ou le cuir chevelu.

Les articles cosmétiques de type lingettes pré-imprégnées jetables, sont bien connus pour leur coté pratique. Ils sont constitués d'une feuille de substrat insoluble dans l'eau, généralement en non-tissé, imprégné d'une composition cosmétique liquide. Ils sont prêts à l'emploi. Le non-tissé est découpé dans un format adapté pour un usage unique et imprégné de la juste dose de produit cosmétique nécessaire.

Conditionnés dans des paquets souples pouvant contenir de quelques articles à plusieurs dizaines d'articles, ils peuvent être facilement transportés et utilisés à peu près partout. Fabriqués à partir de non-tissés, Ils peuvent être facilement produits industriellement à des coûts acceptables, et sous des formes variées adaptées au mieux à leur usage.

Le substrat non-tissé utilisé dans les lingettes est généralement un réseau fibreux dont la densité et la composition en fibre est homogène sur toute son épaisseur, et dont les deux faces du substrat ont par conséquent la même composition en fibre. Les fibres utilisées sont généralement des mélanges de fibres dites hydrophobes, pour la plupart en polypropylène ou en polyester téréphtalate, et de fibres dites hydrophiles, pour la plupart d'origine cellulosique comme le coton ou la viscose. La composition cosmétique est donc uniformément imprégnée dans toute l'épaisseur du substrat et lors de l'utilisation de la lingette sur la peau ou les cheveux, celle-ci est libérée de manière équivalente et indifféremment à travers les deux faces de l'article.

Ainsi, lorsque l'utilisatrice prend en main la lingette et la passe sur le visage, le corps ou les cheveux, son contenu est libéré autant sur la main que sur la zone à traiter. Ceci peut être perçu comme un défaut important, relativement à d'autres modes d'application plus conventionnels. Ainsi, dans le domaine du démaquillage, l'utilisatrice imprègne une seule face de son coton à démaquiller avec son lait démaquillant et ne se souille donc pas les doigts lors du démaquillage. Ce problème est particulièrement crucial pour des produits tels que les produits autobronzants ou les produits de coloration de la peau ou des cheveux.

Pour cette raison il est intéressant de pouvoir mettre au point une lingette imprégnée dont le substrat soit capable de libérer son contenu préférentiellement à travers une de ses deux faces, qui sera avantageusement tournée vers la zone à traiter, au détriment de l'autre.

Des substrats imprégnés uniquement sur une face ont déjà été proposés. Il s'agit de substrats multicouches comprenant une membrane imperméable fixée soit sur une couche d'un substrat fibreux, soit entre deux couches fibreuses, l'une d'entre elles pouvant être imprégnée d'une composition. Typiquement les différentes couches sont d'abord fabriquées séparément puis assemblées dans une deuxième étape. Le brevet EP1066826 décrit par exemple un substrat tricouche comprenant deux feuilles fibreuses externes et un film thermoplastique imperméable pris en sandwich, le tout étant assemblé par des points de thermosoudure. Une première face du substrat peut servir par exemple à absorber le sébum présent sur le front et le nez, l'autre face imprégnée d'une lotion aqueuse sert à rafraîchir la peau.

Des méthodes sont connues également pour assembler un film imperméable sur un substrat fibreux absorbant, via des techniques d'assemblage par thermocollage ou par enduction à chaud.

Ces solutions ne sont pas satisfaisantes car ces substrats ne sont pas agréables à utiliser à cause de leur grande rigidité d'une part et des problèmes de délaminage des couches d'autre part quand ils sont imprégnés avec des liquides contenant des huiles comme les émulsions ou contenant des glycols.

En outre, le mode d'obtention en plusieurs étapes de ces substrats multicouches les rend plus compliqués à réaliser et plus onéreux qu'un substrat fabriqué en une seule étape. Les modes de "convertissage" (imprégnation du substrat, pliage, conditionnement) sont également plus complexes à mettre en oeuvre, car ils nécessitent des techniques d'enduction visant à imprégner sélectivement une seule face, et à utiliser un mode de pliage spécifique.

Dans tous les exemples de réalisation de la demande FR0309234 déposée par la demanderesse le 28/07/03, la couche la plus hydrophile de la structure multicouches est disposée entre deux couches de fibres en matériau hydrophobe. Elle ne peut donc pas être mise eu contact de la surface à traiter.

Le document US 5 620 694 décrit un tampon multicouche comportant une couche de papier et une couche non-tissée à fibres synthétiques.

Le document EP 1 352 950 enseigne un article comportant un substrat et une composition liquide imprégnée au substrat. Ce document ne divulgue pas un substrat ayant deux faces opposées d'hydrophilies distinctes.

Le document WO 03/043551 divulgue un tampon en coton comportant une surface de traitement.

Aussi, est-ce un des objets de l'invention que de réaliser un article cosmétique du type précité qui permette de résoudre en tout ou partie les problèmes discutés ci-avant en référence aux techniques conventionnelles.

C'est en particulier un objet de l'invention que de réaliser un tel article qui soit simple et économique à réaliser et qui soit d'une utilisation pratique et confortable.

D'autres objets encore apparaîtront dans la description détaillée qui suit.

Selon l'invention, ces objets sont atteints en réalisant un article cosmétique à usage unique comprenant un substrat imprégné d'une composition cosmétique liquide aqueuse, ledit substrat étant perméable et comportant au moins un premier groupe de fibres et au moins un second groupe de fibres dont l'hydrophilie est différente de celle des fibres du premier groupe, lesdits premier et second groupes de fibres étant disposés à l'intérieur d'une même couche du substrat et arrangés de telle sorte qu'une première face du substrat présente une hydrophilie sensiblement supérieure à l'hydrophilie d'une seconde face du substrat, opposée à la première, ladite première face étant externe au substrat.

Ainsi, la première face peut être mise au contact de la surface à traiter, en particulier la peau ou les cheveux.

De préférence, le substrat ne comporte qu'une seule couche.

Avec un tel article, le gain en efficacité d'un traitement cosmétique peut être amélioré de façon sensible en permettant une libération en plus grande quantité sur la surface à traiter, du produit dont est imprégné l'article.

La propreté à l'utilisation de l'article est améliorée, notamment dans le cas de produits autobronzants ou de produits de coloration de la peau ou des cheveux.

Les articles cosmétiques selon l'invention peuvent permettre en outre des modes d'application différents de celui des lingettes conventionnelles. En effet, avec les articles selon l'invention, il est possible d'appliquer le produit sur la zone à traiter avec la face la plus hydrophile puis d'essuyer ou d'estomper ensuite le surplus avec la face la moins hydrophile.

Avantageusement, avec un seul et même article, des traitements en deux étapes peuvent être mis en oeuvre en utilisant par exemple pour exfolier la peau, la face la moins hydrophile, à savoir celle qui est le moins apte à libérer le produit cosmétique, puis en utilisant l'autre face, plus douce, pour libérer massivement sur la peau exfoliée le produit imprégné, par exemple un soin apaisant.

Par "perméable" on désigne un substrat apte à être traversé sur toute son épaisseur par ladite composition cosmétique. Ainsi, à l'inverse de certains articles de l'art antérieur auxquels il a été fait référence précédemment, l'article selon l'invention ne comporte pas de couche imperméable susceptible de former barrière à la composition qui l'imprègne.

Bien entendu, le substrat est insoluble dans l'eau, c'est à dire que son intégrité n'est pas affectée de manière sensible par la présence de la composition cosmétique aqueuse qui l'imprègne, et ce pendant au moins toute la durée de vie du produit.

Le fait que les fibres de premier et second groupes soient disposées à l'intérieur d'une même couche du substrat signifie que les deux groupes de fibres sont liés entre eux lors d'un même process de liage. Au final, dans l'épaisseur de la couche, la distribution des deux types de fibres est inhomogène et pourra être selon un profil caractérisé par :
- une majorité de fibres du premier groupe sur la première face de la couche ;
- une majorité de fibres du second groupe sur la seconde face de la couche et, entre les deux faces,
- une quantité relative des fibres du premier groupe variant selon un profil relativement progressif dont la pente est de signe opposé à celle du profil relativement progressif correspondant à la quantité relative de fibres du second groupe.

Une telle structure se différencie des structures à couches séparées dans lesquelles différentes couches sont formées et liées séparément, puis collées entre elles par thermocollage, soudure à chaud ou par ultrasons.

Avantageusement, la première face du substrat a un indice de relarguage sous pression statique (IRSPS1), la deuxième face du substrat ayant un indice de relarguage sous pression statique (IRSPS2), le rapport IRSPS1/IRSPS2 étant supérieur ou égal à 1,5.

### INDICE DE RELARGUAGE SOUS PRESSION STATIQUE (IRSPS):

Le test qui va être décrit ci-après permet de mesurer l'indice de relarguage sous pression statique (IRSPS) des deux faces d'un article imprégné d'un produit cosmétique tel que défini précédemment. L'IRSPS détermine la quantité de liquide imprégné dans le support susceptible d'être libéré par chacune des deux faces du support sous l'effet d'une pression statique, et de voir par là s'il y a une différence de relarguage entre les 2 faces.

La gestuelle habituellement adoptée par la consommatrice lors de l'utilisation de l'article consistant à essuyer sa peau ou ses cheveux avec une première face de l'article, l'autre face dudit article étant en contact avec sa main, il est souhaitable de déterminer la quantité de liquide libérée simultanément par les deux faces du support.

A cet effet, on pèse précisément au centième de gramme près, au moyen d'une balance Mettler Toledo PR5002, deux feuilles de matériau absorbant de type papier essuie-tout, de marque Wypall L30 (référence 7303) fourni par Kimberley-Clark, dont le grammage est 50 g/m². Les feuilles de matériau absorbant doivent être au moins aussi grandes que l'article à tester et, de préférence, un peu plus grandes de sorte qu'elles dépassent du bord extérieur du support d'au moins deux centimètres.

On place la première feuille sur une plaque en verre de dimensions au moins aussi grandes que celles de l'article à tester. On pose l'article à tester sur la première feuille de Wypall L30. On pose par dessus la deuxième feuille de Wypall L30.

On pose sur l'ensemble une plaque métallique de surface au moins égale à la surface de l'article à tester, d'un poids de 3,7 kg. Après une minute on enlève la plaque et on pèse chaque feuille de Wypall L30.

Le poids de liquide qui a imprégné chaque feuille de Wypall L30 est calculé par différence de la masse du Wypall L30 après et avant le test. Ce poids de liquide correspond à la quantité de liquide qui a été libérée par chacune des deux faces du support, notée respectivement IRSPS1 pour la face ayant la plus grande hydrophilie et IRSPS2 pour la face ayant la plus faible hydrophilie.

Le rapport entre IRSPS1 et IRSPS2 indique la différence de relarguage entre les 2 faces de la lingette.

La valeur retenue pour le rapport IRSPS1/IRSPS2 est la valeur moyenne de mesures opérées sur quatre articles.

Avantageusement, le rapport IRSPS1/IRSPS2 va de 1,5 à 15, et de préférence, de 2,5 à 10, et de préférence encore, de 2,5 à 7.

Bien évidemment, lorsque l'article est creux et est configuré notamment sous forme d'un gant, on sépare les deux feuilles qui le constituent et on effectue le test sur les feuilles ainsi séparées.

### LE SUBSTRAT :

Le substrat est fabriqué de préférence selon le procédé de liage par jets d'eau, classiquement utilisés pour la préparation des non-tissés.

La première étape est la constitution des nappes de fibres ayant des hydrophilies différentes. Ces nappes peuvent être préparées selon différentes méthodes connues de l'homme de l'art, soit par cardage à partir de balles de fibres, soit par extrusion de polymère sous la forme de filaments continus de fibres, soit par voie aéro-pneumatique.

La nappe de fibres les plus hydrophiles ainsi que la nappe de fibres les moins hydrophiles sont déposées sur un tapis roulant et acheminées sous des rampes de jets d'eau à haute pression qui lient les fibres entre elles. Ces jets d'eau sous haute pression provoquent un réarrangement des fibres dans les trois directions au sein de la structure fibreuse conduisant ainsi à un enchevêtrement des fibres les liant entre elles sans ajout de liants chimiques. Généralement les nappes de fibres passent sous plusieurs rampes de jets d'eau dont la pression augmente graduellement de la première rampe jusqu'à la dernière. Cette technique est connue sous le nom d'hydroliage.

Le substrat peut par ailleurs être embossé, ajouré, calandré, imprimé ou subir tout type de traitement en fin de ligne. Ainsi, avantageusement, une desdites faces du support, notamment la moins hydrophile, est rendue exfoliante, en particulier par un calandrage à chaud.

Selon ce process particulier, Il est à noter que, bien que formé à partir de deux nappes de fibres, au final, le substrat ne comporte qu'une seule couche. Les risques de délaminage qui ont pu être observés dans des structures multicouches auxquelles il a été fait référence précédemment sont supprimés.

Les fibres les plus hydrophiles peuvent être choisies parmi les fibres de coton, de cellulose, ou de viscose.

Les fibres les moins hydrophiles peuvent être choisies parmi les fibres de polypropylène, polyester, polyamide, ou polyéthylène.

Avantageusement, le substrat est imprégné par ladite composition cosmétique selon un taux allant de 100% à 1000%, de préférence, de 150% et 800%, et de préférence encore, de 150% et 400% en poids de composition par poids de substrat non imprégné.

### COMPOSITION COSMETIQUE :

La composition utilisée selon l'invention pour l'imprégnation du substrat insoluble dans l'eau, contient un milieu aqueux physiologiquement acceptable, c'est-à-dire compatible avec la peau, les muqueuses, les cheveux et le cuir chevelu.

La composition cosmétique liquide peut contenir entre 10% d'eau et 99,9%, et de préférence, entre 30% et 90% d'eau.

Les compositions utilisées selon l'invention peuvent se présenter sous toutes les formes galéniques appropriées pour une application topique, notamment sous forme de solutions aqueuses ou hydroalcooliques, de lotions homogènes ou biphasées, de laits, de gels aqueux ou hydroalcooliques, d'émulsions obtenues par dispersion d'une phase grasse dans une phase aqueuse (H/E) ou inversement (E/H), de suspensions, de microémulsions, de microcapsules, de microparticules, ou encore de dispersions vésiculaires de type ionique (liposomes) et/ou non ionique.

Notamment dans le cas d'une émulsion, la phase grasse peut être présente à hauteur de 0,5 à 80 % en poids, et de préférence de 1 à 50 % en poids par rapport au poids total de la composition cosmétique.

La phase grasse ou phase huileuse contient habituellement au moins une huile. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles d'amande douce, de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearinerie Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R1COOR2 et R1OR2 dans laquelle R1 représente le reste d'un acide gras comportant de 8 à 29 atomes de carbone, et R2 représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12 ou le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfiuoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

Dans le cas ou la composition cosmétique utilisée selon l'invention est une émulsion, les huiles, les émulsionnants et les coémulsionnants utilisés sont choisis parmi ceux classiquement utilisés dans le domaine cosmétique ou dermatologique.

L'émulsionnant et éventuellement le coémulsionnant sont présents, dans la composition, en une proportion allant de 0,3 à 30 % en poids, et de préférence de 0,5 à 20 % en poids par rapport au poids total de la composition.

La composition selon l'invention peut contenir des émulsionnants ioniques ou non ioniques, dont le choix dépend de l'émulsion souhaitée (E/H ou H/E). On peut utiliser comme émulsionnants ceux habituellement utilisés dans le domaine considéré.

Comme émulsionnants, on peut citer par exemple les tensioactifs non ioniques tels que les esters d'acide gras et de polyols, et leurs dérivés oxyalkylénés et notamment oxyéthylénés ; les éthers d'alcools gras et de polyols, et leurs dérivés oxyalkylénés et notamment oxyéthylénés, et leurs mélanges. Quand il s'agit d'esters d'acide gras et de polyols oxyalkylénés ou d'éthers d'alcools gras et de polyols oxyalkylénés, il peut y avoir par exemple de 1 à 150 groupes oxyalkylénés et notamment oxyéthylénés. Comme émulsionnants, on peut citer plus particulièrement le mélange de stéarate de glycéryle et de stéarate de PEG-100, vendu sous le nom d'Arlacel 165 par la société ICI ; les éthers d'alcools gras polyoxyéthylénés comportant de 1 à 100 groupes oxyéthylénés, comme par exemple le ceteareth-12 et le ceteareth-20, ainsi que les mélanges les contenant comme le mélange commercialisé sous la dénomination Emulgade CM par la société Henkel (mélange de isononanoate de cétéaryle, ceteareth-20, alcool cétéarylique, stearate de glyceryle, glycérine, ceteareth-12 et palmitate de cétyle). Les émulsionnants cités ci-dessus sont utilisés pour la préparation d'émulsions H/E.

On peut ajouter aussi dans la composition de l'invention des tensioactifs non ioniques, anioniques, amphotères ou zwitterioniques, favorisant l'élimination du maquillage et des impuretés, et pouvant rendre la composition moussante. Il peut s'agir notamment de tensioactifs moussants. Comme tensioactifs de ce type, on peut citer par exemple :
(1) parmi les tensioactifs non ioniques, les polymères blocs oxyéthylénés oxypropylénés tels que le Poloxamer 184 (nom CTFA) ; les alkylpolyglycosides et notamment les alkylpolyglucosides (APG) ayant un groupe alkyle comportant de 6 à 30 atomes de carbone (alkyl-C6-C30 polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel ;
(2) parmi les tensioactifs anioniques, les alkylsulfates, les alkyl éther sulfates et leurs sels, notamment leurs sels de sodium, comme le mélange de Sodium Laureth Sulfate / Magnésium Laureth Sulfate / Sodium Laureth-8 Sulfate / Magnésium Laureth-8 Sulfate, vendu sous le nom de Texapon ASV par la société Henkel ; le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel ; l'alkyl (C12-C14) éther (9 OE) sulfate d'ammonium commercialisé sous la dénomination RHODAPEX AB/20 par la société Rhodia Chimie ;
(3) parmi les tensioactifs amphotères ou zwitterioniques, les dérivés alkylamido alkylamines tels que le N-cocoyl-N-carboxyméthoxyéthyl-N-carboxyméthyl-éthylènediamine N-di-sodique (nom CTFA : Disodium cocoampho-diacetate) commercialisé en solution aqueuse saline sous la dénomination MIRANOL C2M CONC NP par la société Rhodia Chimie ; le N-cocoyl-N-hydroxyéthyl-N-carboxyméthyl-éthylènediamine N-sodique (nom CTFA : sodium cocamphoacetate) et le mélange d'éthanolamides d'acide de coco (nom CTFA : Cocamide DEA).

La composition peut comprendre aussi un mélange de ces tensioactifs.

La composition cosmétique peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, l'hexylène glycol, le propylène glycol, les polyéthylène glycols tels que le PEG-8 ; le sorbitol ; les sucres tels que le glucose, le fructose, le maltose, le lactose, le sucrose ; et leurs mélanges.

La composition utilisée pour imprégner le substrat peut comprendre en outre les adjuvants classiquement mis en oeuvre dans les domaines considérés, comme par exemple les solvants organiques, les agents solubilisants, les agents épaississants et gélifiants hydrophiles ou lipophiles, les adoucissants, les antioxydants, les opacifiants, les agents stabilisants, les agents moussants, les charges, les chélateurs, les parfums, les filtres, les huiles essentielles, les matières colorantes, les pigments, les actifs hydrophiles ou lipophiles, les vésicules lipidiques encapsulant éventuellement un ou plusieurs actifs, ou tout autre ingrédient habituellement utilisé en cosmétique ou dermatologie.

Elle peut éventuellement contenir aussi des conservateurs autres que ceux cités ci-dessus. Les quantités des différents constituants des compositions selon l'invention sont celles classiquement utilisées dans les domaines considérés.

Comme actifs, on peut citer par exemple, sans que cette liste ne soit limitative, les actifs antiséborrhéiques qui permettent un nettoyage de l'excédent de sebum sur la peau, et les agents antimicrobiens qui éliminent de la peau les microorganismes qui y sont éventuellement présents, et leurs mélanges.

Comme actifs antiséborrhéiques, on peut citer par exemple le soufre et les dérivés soufrés, le peroxyde de benzoyle, les dérivés de zinc tels que le sulfate de zinc et l'oxyde de zinc, le chlorure d'aluminium, le disulfure de sélénium, les vitamines B et notamment le panthénol (vitamine B5) et la niacinamide (vitamine B6 ou PP), et leurs mélanges.

Comme antimicrobiens, on peut citer par exemple les actifs suivants : dérivés de □-lactam, dérivés de quinolone, ciprofloxacine, norfloxacine, tétracycline et ses sels (chlorhydrate), érythromycine et ses sels (de zinc, estolate, stéarate), amikacine et ses sels (sulfate), 2,4,4'-trichloro-2'-hydroxy diphenyl éther (triclosan), 3,4,4'-trichlorobanilide (tricarban), phénoxyéthanol, phénoxypropanol, phénoxyisopropanol, doxycycline et ses sels (chlorhydrate), capréomycine et ses sels (sulfate), chlorhexidine et ses sels (gluconate, chlorhydrate), chlorotétracycline et ses sels (chlorhydrate), oxytétracycline et ses sels (chlorhydrate), clindamycine et ses sels (chlorhydrate), éthambutol et ses sels (chlorhydrate), hexamidine et ses sels (iséthionate), métronidazole et ses sels (chlorhydrate), pentamidine et ses sels (chlorhydrate), gentamicine et ses sels (sulfate), kanamycine et ses sels (sulfate), linéomycine et ses sels (chlorhydrate), méthacycline et ses sels (chlorhydrate), méthenamine et ses sels (hippurate, mandelate), minocycline et ses sels (chlorhydrate), néomycine et ses sels (sulfate), netilmicine et ses sels (sulfate), paromomycine et ses sels (sulfate), streptomycine et ses sels (sulfate), tobramycine et ses sels (sulfate), miconazole et ses sels (chlorhydrate), amanfadine et ses sels (sulfate, chlorhydrate), octopirox, parachlorometaxylenol, nystatine, tolnaftate, zinc pyrithione, clotrimazole, acide salicylique, acide n-octanoyi-5 salicylique (ou acide capryloyl-salicylique), peroxyde de benzoyle, acide 3-hydroxybenzoique, acide glycolique, acide lactique, acide 4-hydroxy-benzoique, acide acétylsalicylique, acide 2-hydroxybutanoique, acide 2-hydroxypentanoique, acide 2-hydroxyhexanoique, acide phytique , acide N-acetyl-L-cysteine, acide lipoique, acide azélaïque, acide arachidonique, ibuprofen, naproxen, hydrocortisone, acetominophen, resorcinol, chlorhydrate de lidocaine, sulfate de néocycine, octoxyglycérine, octanoylglycine (ou capryloylglycine), caprylylglycol (1,2-octanediol), acide 10-hydroxy-2-décanoique, et leurs mélanges. Les agents antimicrobiens préférés sont le 2,4,4'-trichloro-2'-hydroxy diphenyl éther, le 3,4,4'-trichlorobanilide, le phénoxyéthanol, le phénoxypropanol, le phénoxyisopropanol, la chlorhexidine et ses sels, l'octopirox, le zinc pyrithione, l'acide salicylique, l'acide n-octanoyl-5 salicylique, le peroxyde de benzoyle, l'acide 3-hydroxybenzoique, l'acide glycolique, l'acide lactique, l'acide 4-hydroxybenzoique, l'acide acétylsalicylique, l'acide 2-hydroxybutanoique, l'acide 2-hydroxypentanoique, l'acide 2-hydroxyhexanoique, l'acide phytique , l'acide N-acetyl-L-cysteine, l'acide lipoique, l'acide azélaïque, l'acide arachidonique, l'octoxyglycérine, l'octanoylglycine, le caprylylglycol, l'acide 10-hydroxy-2-décanoique, et leurs mélanges.

Comme gélifiants hydrophiles, on peut citer en particulier les polymères carboxyvinyliques comme les carbomer ; les copolymères acryliques modifiés tels que les copolymères d'acrylates/alkylacrylates comme les produits commercialisés sous les dénominations Pemulen par la société Goodrich ; les polyacrylamides comme le produit commercialisé sous la dénomination Sepigel 305 par la société Seppic, ou le poly(acide 2-acrylamido 2-méthylpropane sulfonique) commercialisé par la société Hoechst sous la dénomination commerciale « Hostacerin AMPS » (nom CTFA : ammonium polyacryldimethyl-tauramide) ; les polysaccharides, notamment les dérivés cellulosiques et les gommes naturelles comme lagomme de xanthane ou la gomme guar ; et les argiles. Comme gélifiants lipophiles, on peut citer les argiles modifiées comme les bentones, les sels métalliques d'acides gras, la silice hydrophobe, les polyéthylènes, et leurs mélanges. On peut aussi utiliser comme gélifiants, les polymères à fonction hydrophobe, tels que les polysaccharides à chaîne hydrophobe comme les gommes de guar quaternisées.

Les compositions selon l'invention sont préparées selon les techniques bien connues de l'homme de l'art du domaine.

Selon un mode particulier de réalisation de l'invention, la composition se présente sous forme d'une émulsion PIT.

Cette technique d'obtention d'émulsion H/E est, dans son principe, bien connue de l'homme de l'art et est notamment décrite dans les articles "Phase Inversion Emusification", par Th. Förster et al, paru dans Cosmetics & Toiletries, vol. 106, Decembre 1991, pp 49-52, «Application of the phase-inversion-température method to the emulsification of cosmetics» par T. MITSUI et al, paru dans American Cosmetics and Perumery, vol 87, Decembre 1972.

Cette technique permet d'obtenir des émulsions H/E dites "ultrafines", dans lesquelles la taille moyenne des globules constituant la phase grasse est comprise dans des limites bien déterminées, à savoir entre 50 et 1000 nm. Ces émulsions sont extrêmement fluides et sont particulièrement bien appropriées pour imprégner des substrats insolubles dans l'eau afin de constituer des articles ou lingettes de nettoyage.

Selon un mode particulier de réalisation, l'émulsion PIT est préparée sous forme concentrée puis diluée, généralement juste avant l'imprégnation, par une à neuf parties d'une phase aqueuse pouvant également contenir tout ou une partie des conservateurs préalablement dissous.

La viscosité de la composition cosmétique utilisée dans l'article selon l'invention est de préférence inférieure à 1500 mPa.s et, de préférence encore, inférieure à 1000 mPa.s. La viscosité est mesurée à la température ambiante (environ 25°C) avec un appareil RHEOMAT RM 180.

L'article cosmétique selon l'invention peut être configuré sous forme d'une lingette, d'une compresse, d'un gant, d'une moufle, d'un chausson, d'un doigtier, d'un bonnet, d'une coiffe ou d'un masque en forme de tout ou partie du visage. Dans ce dernier cas, le masque pourra être conçu pour le visage complet ou spécifiquement pour le haut ou le bas du visage uniquement.

Dans le cas d'un gant ou d'un doigtier à utiliser classiquement pour traiter une surface du corps (peau ou cheveux) autre que la main, la face ayant l'hydrophilie la plus élevée sera avantageusement positionnée vers l'extérieur de l'article, en tant que surface servant à déposer le produit imprégné.

On pourra utiliser également un article configuré sous forme d'un gant, d'un doigtier ou d'un chausson pour traiter tout ou partie de la main ou du pied, notamment dans le cas d'un traitement adoucissant, hydratant, ou anti-taches. Dans ce cas, la surface la plus hydrophile sera interne au gant ou au chausson.

De même, dans le cas d'un article configuré notamment sous forme d'une coiffe, d'un bonnet ou d'une charlotte, la face ayant l'hydrophilie la plus élevée sera avantageusement positionnée vers l'intérieur de l'article, en tant que surface servant à déposer le produit imprégné.

Dans le cas d'un masque imprégné pour le soin du visage, la face ayant l'hydrophilie la plus élevée sera avantageusement positionnée contre le visage.

La composition cosmétique utilisée dans l'article selon l'invention peut être une composition capillaire, notamment pour le nettoyage ou la coloration/décoloration des cheveux, ou une composition pour la peau ou ses muqueuses, notamment une composition de soin, une composition autobronzante, une composition de démaquillage, une composition à effet desquamant, ou une composition de coloration.

Selon un autre aspect de la présente invention on réalise un article cosmétique à usage unique comprenant au moins un substrat imprégné d'une composition cosmétique liquide, ledit substrat étant perméable et comportant au moins un premier groupe de fibres et au moins un second groupe de fibres dont l'hydrophilie est différente de celle des fibres du premier groupe, lesdits premier et second groupes de fibres étant disposés à l'intérieur d'une même couche du substrat de telle sorte qu'une première face du substrat, externe à ce dernier, présente une hydrophilie supérieure à l'hydrophilie d'une seconde face du substrat, opposée à la première, ledit article comportant au moins une portion creuse ou concave, ladite portion creuse ou concave étant destinée :
i) soit à recevoir une surface à traiter de manière à mettre cette dernière en contact avec une surface interne de l'article ;
ii) soit à recevoir tout ou partie d'une main, en vue d'amener une surface externe de l'article en engagement avec une surface à traiter.

Un tel article creux ou concave peut être configuré sous forme d'un gant, d'une moufle, d'un chausson, d'un doigtier, d'un bonnet, d'une coiffe ou d'une charlotte.

Un tel article creux peut être obtenu par assemblage le long de leurs bords respectifs d'au moins "deux feuilles", ou par repliement d'une feuille sur elle même puis par fixation sur eux mêmes des bords de la feuille ainsi repliée.

Lorsque la portion creuse ou concave est destinée à recevoir tout ou partie d'une main, en vue d'amener une surface externe de l'article en engagement avec une surface à traiter, la face la plus hydrophile est à l'extérieur de la portion creuse ou concave.

Inversement, lorsque la portion creuse ou concave est destinée à recevoir une surface à traiter, lorsqu'il s'agit notamment d'un gant, d'une moufle, d'un chausson, d'un doigtier, d'un bonnet, d'une coiffe ou d'une charlotte, la face la plus hydrophile est à l'intérieur de la portion creuse ou concave.

De préférence, le substrat est monocouche.

### EXEMPLE 1 : Gant Nettoyant capillaire :

La composition nettoyante décrite ci-dessous est imprégnée à 200% sur un non-tissé Sandler ref. 03/02/1001.

| **Ingrédients :** | **% massique :** |
|---|---|
| EAU | Qsp 100 |
| ALCOHOL DENAT. (93.1 %) / AQUA | 20 % |
| DISODIUM COCOAMPHODIACETATE | 0.15 % |
| HYDROGENATED POLYISOBUTENE | 5 % |
| PARFUM | 0.2 % |

Le non-tissé Sandler ref. 03/02/1001 est obtenu par hydroliage et composé de 100% de fibres de viscose sur une face et de 100% de fibres de Polypropylene (PP) sur l'autre face. Il est découpé à la forme d'une moufle ou d'un gant dont les faces PP sont soudées vers l'intérieur par les bords de façon à délimiter une poche dans laquelle introduire la main.

La présence de fibres de PP, de par leur caractère fusible permet de souder le gant, et de par leur caractère hydrophobe permet à la main de ne pas ou très peu être en contact avec le jus imprégné sur le substrat.

La présence de fibres de viscose à l'extérieur du gant permet une bonne imprégnation du jus et un relarguage optimal de la lotion sur la surface à nettoyer (peau ou cheveux).

Pour l'article selon cet exemple, le rapport IRSPS1/IRSPS2 est de 8,4.

### EXEMPLE 2 : Lingette exfoliation mécanique/Peeling chimique :

La composition exfoliante décrite ci-dessous est imprégnée à 300% sur un non-tissé Jacob Holm ref. Rough & Soft 107103/003A.

| **Ingrédients :** | **% massique** |
|---|---|
| EAU | Qsp 100 |
| ALCOHOL DENAT. (93.1 %) / AQUA | 5 % |
| GLYCERINE | 3 % |
| CONSERVATEURS | 0.5 % |
| ACIDE GLYCOLIQUE | 1 % |
| PARFUM | 0.2 % |

Le non-tissé Jacob Holm ref. Rough & Soft 107103/003A est obtenu par hydroliage et est composé de 100% d'un mélange de fibres de viscose et de polyester téréphtalate sur une face et de 100% de fibres de Polypropylène (PP) sur l'autre face.

Ces fibres de PP confèrent à la face du substrat un caractère rugueux propice à une exfoliation mécanique lors du passage de cette face sur la peau. La lotion peeling est ensuite appliquée en passant sur la peau l'autre face douce et hydrophile.

Pour l'article selon cet exemple, le rapport IRSPS1/IRSPS2 est de 1,75.

### EXEMPLE 3 : Lingette exfoliation mécanique / Lotion apaisante :

La composition apaisante décrite ci-dessous est imprégnée à 300% sur un non-tissé PGI Duralace 7163, 80gsm Cartex Blue.

| **Ingrédients :** | **% massique :** |
|---|---|
| EAU | Qsp 100 |
| ALLANTOÏNE | 0.15 % |
| GLYCERINE | 5% |
| PEG-8 | 3% |
| CONSERVATEURS | 0.3 % |
| GLYCYRRHETINATE DE POTASSIUM | 0.1 % |
| PARFUM | 0.1 % |

Le non-tissé PGI Duralace 7163, 80gsm Cartex Blue est obtenu par hydroliage et est composé de 100% d'un mélange de fibres de viscose et de polyester téréphtalate sur une face et de 100% de fibres de Polypropylène (PP) de fort denier sur l'autre face.

Les fibres de PP confèrent à la face du substrat un caractère rugueux propice à une exfoliation mécanique lors du passage de cette face sur la peau. La lotion apaisante est ensuite appliquée en passant sur la peau l'autre face douce et hydrophile.

La lingette selon cet exemple peut également être utilisée comme un masque en la laissant posée sur le visage, la face douce et hydrophile tournée vers le visage.

Pour l'article selon cet exemple, le rapport IRSPS1/IRSPS2 est de 2,1.

### EXEMPLE 4 : Lingette démaquillante:

La composition de démaquillage décrite ci-dessous est imprégnée à 300% sur un non-tissé Sandler ref. 03/02/1001.

| **Ingrédients :** | **% massique :** |
|---|---|
| WATER | Qsp 100 |
| XANTHAN GUM | 0.1% |
| GLYCERYLSTEARATE | 0.25% |
| ISOPROPYL PALMITATE | 1% |
| CETYL ALCOHOL | 0.15% |
| GLYCERIN | 3% |
| CONSERVATEURS | 0.45% |
| GLYCINE SOJA (SOYBEAN) OIL | 0.05% |
| PEG-100 STEARATE | 0.25% |
| FRAGRANCE | 0.05% |
| PRUNUS AMYGDALUS DULCIS (SWEET ALMOND) OIL | 1.2% |

Le non-tissé Sandler ref. 03/02/1001 est obtenu par hydroliage et composé de 100% de fibres de viscose sur une face et de 100% de fibres de Polypropylène (PP) sur l'autre face.

La présence de fibres de viscose sur une première face du substrat permet une bonne imprégnation du jus et un relarguage optimal du lait démaquillant pour un meilleur démaquillage.

La présence de fibres de PP sur la deuxième face du substrat permet un relarguage minimum du lait démaquillant par cette face, qui sera avantageusement utilisée pour éliminer les résidus du démaquillage.

Pour l'article selon cet exemple, le rapport IRSPS1/IRSPS2 est de 2 ,65.

### EXEMPLE 5 : Lingette exfoliante/démaquillante :

La composition de démaquillage décrite ci-dessous est imprégnée à 300% sur un non-tissé Jacob Holm ref. Rough & Soft 107103/003A.

| **Ingrédients :** | **% massique :** |
|---|---|
| WATER | Qsp 100 |
| DICAPRYLYL ETHER | 3% |
| ETHYLHEXYL PALMITATE | 3% |
| CETEARYL ALCOHOL | 0.31% |
| CETEARETH-12 | 0.62% |
| CETEARETH-20 | 1.52% |
| PEG-4 DILAURATE | 0.08% |
| GLYCERIN | 5% |
| MINERAL OIL | 5% |
| CONSERVATEURS | 0.15% |
| FRAGRANCE | 0.3% |

Le non-tissé Jacob Holm ref. Rough & Soft 107103/003A est obtenu par hydroliage et est composé de 100% d'un mélange de fibres de viscose et de polyester téréphtalate sur une face et de 100% de fibres de Polypropylène (PP) sur l'autre face.

La lotion démaquillante est d'abord appliquée en passant sur la peau la face douce et hydrophile permettant un relarguage optimal et donc une meilleure efficacité de démaquillage. Les fibres de polypropylene confèrent à la face la moins hydrophile du substrat un caractère rugueux propice à une exfoliation légère pour un nettoyage en profondeur.

Pour l'article selon cet exemple, le rapport IRSPS1/IRSPS2 est de 4.

### EXEMPLE 6 : Lingette autobronzante :

La composition autobronzante décrite ci-dessous est imprégnée à 400% sur un non-tissé Jacob Holm ref. Rough & Soft 107103/003A.

| **Ingrédients :** | **% massique :** |
|---|---|
| EAU | Qsp 100 |
| DIHYDROXY ACETONE | 5 % |
| CONSERVATEURS | 0,5% |
| PROPYLENE GLYCOL | 25 % |
| BEHENETH10 | 2,5% |
| CYCLOPENTASILOXANE | 15 % |
| LAURETH-4 | 2.5% |
| ISOPROPYL MYRISTATE | 6% |

Le non-tissé Jacob Holm ref. Rough & Soft 107103/003A est obtenu par hydroliage et est composé de 100% d'un mélange de fibres de viscose et de polyester téréphtalate sur une face et de 100% de fibres de Polypropylène (PP) de fort denier sur l'autre face.

Les fibres de PP confèrent à la face du substrat un caractère rugueux propice à une exfoliation mécanique lors du passage de cette face sur la peau. Cette exfoliation permet d'éliminer les cellules mortes et de préparer en quelque sorte la peau. La lotion autobronzante est ensuite appliquée en passant sur la peau l'autre face douce et hydrophile.

Pour l'article selon cet exemple, le rapport IRSPS1/IRSPS2 est de 2,93.

### EXEMPLE 7 : masque de soin :

La composition de soin décrite ci-dessous est imprégnée à 600% sur un non-tissé Sandler ref. 03/02/1001.

| **Ingrédients** | **% massique** |
|---|---|
| EAU | Qsp 100 |
| XANTHAN GUM | 0.15 % |
| SODIUM MYRISTOYL GLUTAMATE | 0.05% |
| CYCLOPENTASILOXANE | 0,4 % |
| CONSERVATEURS | 0.2% |
| BUTYLENE GLYCOL | 10% |
| GLYCERINE | 3% |
| PARFUM | 0.2 % |

Le non-tissé Sandler ref. 03/02/1001 est obtenu par hydroliage et composé de 100% de fibres de viscose sur une face et de 100% de fibres de Polypropylène (PP) sur l'autre face.

La présence de fibres de viscose à l'extérieur du masque permet de concentrer la majeure partie de la composition de soin sur cette face et de la relarguer de manière optimale sur le visage. De ce fait on peut imprégner le masque d'une moindre quantité de composition de soin, tout en obtenant un aussi bon effet. L'une ou l'autre des faces peut être avantageusement imprimée ou faite à partir de fibres colorées pour bien discerner la face à appliquer sur le visage.

Pour l'article selon cet exemple, le rapport IRSPS1/IRSPS2 est de 1,75.

## Revendications

1. Article cosmétique à usage unique comprenant un substrat imprégné d'une composition cosmétique liquide aqueuse, ledit substrat étant perméable et comportant au moins un premier groupe de fibres et au moins un second groupe de fibres dont l'hydrophilie est différente de celle des fibres du premier groupe, lesdits premier et second groupes de fibres étant disposés à l'intérieur d'une même couche du substrat et étant arrangés de telle sorte qu'une première face du substrat présente une hydrophilie sensiblement supérieure à l'hydrophilie d'une seconde face du substrat, opposée à la première, ladite première face étant externe au substrat, le substrat étant monocouche.

2. Article cosmétique selon la revendication 1 **caractérisé en ce que** la première face du substrat a un indice de relarguage sous pression statique (IRSPS1), la deuxième face du substrat ayant un indice de relarguage sous pression statique (IRSPS2), le rapport IRSPS1/IRSPS2 étant supérieur ou égal à 1,5.

3. Article cosmétique selon la revendication 2 **caractérisé en ce que** le rapport IRSPS1/IRSPS2 va de 1,5 à 15, et de préférence, de 2,5 à 10, et de préférence encore, de 2,5 à 7.

4. Article cosmétique selon l'une quelconque des revendications 1 à 3 **caractérisé en ce que** le substrat est obtenu par hydroliage d'une première nappe desdites fibres du premier groupe et d'une seconde nappe desdites fibres du second groupe.

5. Article cosmétique selon l'une quelconque des revendications 1 à 4 **caractérisé en ce que** le substrat est imprégné par ladite composition cosmétique selon un taux allant de 100% à 1000%, de préférence, de 150% et 800%, et de préférence encore, de 150% et 400% en poids de composition par poids de substrat non imprégné.

6. Article cosmétique selon l'une quelconque des revendications précédentes **caractérisé en ce que** la composition cosmétique liquide contient entre 10% d'eau et 99,9%, et de préférence, entre 30% et 90% d'eau.

7. Article cosmétique selon l'une quelconque des revendications qui précèdent **caractérisé en ce que** la composition cosmétique liquide comprend une phase grasse.

8. Article cosmétique selon la revendication précédente **caractérisé en ce que** la phase grasse représente de 0,5 à 80 % en poids, et de préférence de 1 à 50 % en poids par rapport au poids total de la composition cosmétique.

9. Article cosmétique selon la revendication 7 ou 8 **caractérisé en ce que** la phase grasse est choisie parmi les huiles hydrocarbonées d'origine animale, les huiles hydrocarbonées d'origine végétale, les esters et les éthers de synthèse, notamment d'acides gras, les hydrocarbures linéaires ou ramifiés, d'origine minérale ou synthétique, les alcools gras ayant de 8 à 26 atomes de carbone, les alcools gras alcoxylés et notamment éthoxylés, les huiles fluorées partiellement hydrocarbonées et/ou siliconées, les huiles de silicone, et leurs mélanges.

10. Article cosmétique selon l'une quelconque des revendications qui précèdent **caractérisé en ce que** la composition cosmétique contient en outre un ou plusieurs composés choisis parmi les solvants organiques, les agents solubilisants, les agents épaississants et gélifiants hydrophiles ou lipophiles, les adoucissants, les antioxydants, les opacifiants, les agents stabilisants, les agents moussants, les charges, les chélateurs, les parfums, les filtres, les huiles essentielles, les matières colorantes, les pigments, les actifs hydrophiles ou lipophiles, les vésicules lipidiques encapsulant éventuellement un ou plusieurs actifs.

11. Article cosmétique selon l'une quelconque des revendications précédentes **caractérisé en ce que** la composition cosmétique contient, outre de l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, l'hexylène glycol, le propylène glycol, les polyéthylène glycols tels que le PEG-8 ; le sorbitol ; les sucres tels que le glucose, le fructose, le maltose, le lactose, le sucrose ; et leurs mélanges.

12. Article cosmétique selon l'une quelconque des revendications 1 à 11 **caractérisé en ce que** la composition cosmétique présente une viscosité inférieure à 1500 mPa.s et, de préférence, inférieure à 1000 mPa.s.

13. Article cosmétique selon l'une quelconque des revendications précédentes **caractérisé en ce que** les fibres du premier groupe sont choisies parmi les fibres de coton, de cellulose, ou de viscose.

14. Article cosmétique selon l'une quelconque des revendications précédentes **caractérisé en ce que** les fibres du second groupe sont choisies parmi les fibres de polypropylène, polyester, polyamide, ou polyéthylène.

15. Article cosmétique selon l'une quelconque des revendications précédentes **caractérisé en ce qu'**une desdites faces du support, notamment la seconde, est rendue exfoliante, notamment par un calandrage à chaud.

16. Article cosmétique selon l'une quelconque des revendications qui précèdent **caractérisé en ce qu'**il est configuré sous forme d'une lingette, d'une compresse, d'un gant, d'une moufle, d'un doigtier, d'un chausson, d'un bonnet, d'une coiffe ou d'un masque en forme de tout ou partie du visage.

17. Article cosmétique selon l'une quelconque des revendications précédentes **caractérisé en ce que** la composition cosmétique est une composition capillaire, notamment de coloration ou pour le nettoyage des cheveux, ou une composition pour la peau ou ses muqueuses, notamment une composition de soin, une composition autobronzante, une composition de démaquillage, une composition à effet desquamant, ou une composition de coloration.

18. Article cosmétique à usage unique comprenant au moins un substrat imprégné d'une composition cosmétique liquide, ledit substrat étant perméable et comportant au moins un premier groupe de fibres et au moins un second groupe de fibres dont l'hydrophilie est différente de celle des fibres du premier groupe, lesdits premier et second groupes de fibres étant disposés à l'intérieur d'une même couche du substrat et arrangés de telle sorte qu'une première face du substrat, externe à ce dernier, présente une hydrophilie supérieure à l'hydrophilie d'une seconde face du substrat, opposée à la première, ledit article comportant au moins une portion creuse ou concave, ladite portion creuse ou concave étant destinée :
iii) soit à recevoir une surface à traiter ;
iv) soit à recevoir tout ou partie d'une main en vue d'amener l'article en engagement avec une surface à traiter,
le substrat étant monocouche.

19. Article cosmétique selon la revendication 18 **caractérisé en ce qu'**il est configuré sous forme d'un gant, d'une moufle, d'un doigtier, d'un chausson, d'un bonnet, d'une coiffe ou d'une charlotte.

20. Article cosmétique selon la revendication 18 ou 19 **caractérisé en ce que** la portion creuse ou concave est destinée à recevoir tout ou partie d'une main en vue d'amener l'article en engagement avec une surface à traiter, la première face étant à l'extérieur de la portion creuse ou concave.

21. Article cosmétique selon la revendication 18 ou 19 **caractérisé en ce que** la portion creuse ou concave est destinée à recevoir une surface à traiter, la première face étant à l'intérieur de la portion creuse ou concave.

## Patentansprüche

1. Kosmetikartikel zur einmaligen Verwendung, umfassend ein Substrat imprägniert mit einer wässrigen, flüssigen kosmetischen Zusammensetzung, wobei das Substrat permeabel ist und mindestens eine erste Fasergruppe und mindestens eine zweite Fasergruppe umfasst, deren Hydrophilie sich von der der Fasern der ersten Gruppe unterschiedet, und sich die erste und zweite Fasergruppe in derselben Schicht des Substrats befinden und derart angeordnet sind, dass eine erste Seite des Substrats eine im Wesentlichen höhere Hydrophilie als die Hydrophilie einer zweiten Seite aufweist, die der ersten Seite gegenüberliegt, wobei die erste Seite extern vom Substrat ist und das Substrat aus einer Schicht besteht.

2. Kosmetikartikel nach Anspruch 1, **dadurch gekennzeichnet, dass** die erste Seite des Substrats eine Freisetzungsrate unter statischem Druck (IRSPS1), und die zweite Seite eine Freisetzungsrate unter statischem Druck (IRSPS2) hat, wobei das Verhältnis IRSPS1/IRSPS2 größer oder gleich 1,5 ist.

3. Kosmetikartikel nach Anspruch 2, **dadurch gekennzeichnet, dass** das Verhältnis IRSPS1/IRSPS2 von 1,5 bis 15, und vorzugsweise von 2,5 bis 10, und noch vorzugsweiser von 2,5 bis 7 ist.

4. Kosmetikartikel nach einem der Ansprüche 1 bis 3, **dadurch gekennzeichnet, dass** das Substrat durch Wasserstrahlverwirbelung eines ersten Vlieses der Fasern der ersten Gruppe und eines zweiten Vlieses der Fasern der zweiten Gruppe erlangt wird.

5. Kosmetikartikel nach einem der Ansprüche 1 bis 4, **dadurch gekennzeichnet, dass** das Substrat mit der kosmetischen Zusammensetzung in einer Rate von 100 % bis 1000 %, vorzugsweise von 150 % und 800 %, und noch vorzugsweiser von 150 % und 400 % in Gewicht der Zusammensetzung pro Gewicht nicht imprägnierten Substrats.

6. Kosmetikartikel nach einem der vorherigen Ansprüche, **dadurch gekennzeichnet, dass** die flüssige kosmetische Zusammensetzung zwischen 10 % und 99,9 % Wasser, und vorzugsweise zwischen 30 % und 90 % Wasser enthält.

7. Kosmetikartikel nach einem der vorherigen Ansprüche, **dadurch gekennzeichnet, dass** die flüssige kosmetische Zusammensetzung eine fette Phase umfasst.

8. Kosmetikartikel nach dem vorherigen Anspruch, **dadurch gekennzeichnet, dass** die fette Phase 0,5 bis 80 Gew.-% und vorzugsweise 1 bis 50 Gew.-% in Bezug auf das Gesamtgewicht der kosmetischen Zusammensetzung entspricht.

9. Kosmetikartikel nach Anspruch 7 oder 8, **dadurch gekennzeichnet, dass** die fette Phase unter den Kohlenwasserstoffölen tierischen Ursprungs, den Kohlenwasserstoffölen pflanzlichen Ursprungs, den synthetisierten Estern und Ethern, besonders Fettsäuren, den linearen oder verzweigten Kohlenwasserstoffen mineralen oder synthetischen Ursprungs, den fetten Alkoholen mit 8 bis 26 Kohlenstoffatomen, den fetten Alkoxyl- und besonders Ethoxylalkoholen, den teilweisen fluorierten oder silikonisierten Kohlenwasserstoffölen, den Silikonölen und deren Mischungen gewählt ist.

10. Kosmetikartikel nach einem der vorherigen Ansprüche, **dadurch gekennzeichnet, dass** die kosmetische Zusammensetzung u.a. eine oder mehrere Zusammensetzungen enthält, die unter den organischen Lösungsmitteln, den Lösungsvermittlern, den hydrophilen oder lipophilen Verdickungs- und Geliermitteln, den Weichmachern, den Antioxidansen, den Verdunklungsmitteln, den Stabilisierungsmitteln, den Schäumungsmitteln, den Füllstoffen, den Chelaten, den Parfüms, den Filtern, den ätherischen Ölen, den Färbstoffen, den Pigmenten, den hydrophilen oder lipophilen Wirkstoffen, den lipiden Bläschen, die einen oder mehrere Wirkstoffe einkapseln können, gewählt ist.

11. Kosmetikartikel nach einem der vorherigen Ansprüche, **dadurch gekennzeichnet, dass** die kosmetische Zusammensetzung außer dem Wasser ein oder mehrere Lösungsmittel enthält, die unter den unteren Alkoholen mit 1 bis 6 Kohlenstoffatomen, wie z. B. Ethanol; den Polyolen, wie z. B. Glyzerin; den Glykolen, wie z. B. Butylenglykol, Isoprenglykol, Hexylenglykol, Propylenglykol, die Polyethylenglykole, wie z. B. PEG-8; das Sorbitol; die Zucker, wie z. B. die Glykose, die Fruktose, die Maltose, die Laktose, die Saccharose; und deren Mischungen gewählt sind.

12. Kosmetikartikel nach einem der Ansprüche 1 bis 11, **dadurch gekennzeichnet, dass** die kosmetische Zusammensetzung eine Viskosität unter 1500 mPa.s und vorzugsweise unter 1000 mPa.s aufweist.

13. Kosmetikartikel nach einem der vorherigen Ansprüche, **dadurch gekennzeichnet, dass** die Fasern der ersten Gruppe unten den Baumwollfasern, Zellulosefasern oder Viskosefasern ausgewählt sind.

14. Kosmetikartikel nach einem der vorherigen Ansprüche, **dadurch gekennzeichnet, dass** die Fasern der zweiten Gruppe unter den Polypropylen-, Polyester-, Polyamid- oder Polyethylenfasern gewählt sind.

15. Kosmetikartikel nach einem der vorherigen Ansprüche, **dadurch gekennzeichnet, dass** eine der Seiten des Trägers, besonders die zweite Seite, durch Heißkalandrierung hautabreibend gemacht wird.

16. Kosmetikartikel nach einem der vorherigen Ansprüche, **dadurch gekennzeichnet, dass** er in Form eines Kosmetiktuchs, einer Kompresse, eines Handschuhs, eines Fäustlings, eines Däumlings, eines Füßlings, einer Haube, einer Abdeckung oder einer Maske mit der Form des gesamten oder teilweisen Gesichts konfiguriert ist.

17. Kosmetikartikel nach einem der vorherigen Ansprüche, **dadurch gekennzeichnet, dass** die kosmetische Zusammensetzung eine kapillare Zusammensetzung, besonders zur Färbung oder zur Reinigung der Haare, oder eine Zusammensetzung für die Haut oder ihre Schleimhäute, besonders eine Pflegezusammensetzung, eine selbstbräunende Zusammensetzung, eine Abschminkzusammensetzung, eine Zusammensetzung mit schälender Wirkung oder eine Färbungszusammensetzung ist.

18. Kosmetikartikel zur einmaligen Verwendung, umfassend mindestens ein Substrat imprägniert mit einer flüssigen kosmetischen Zusammensetzung, wobei das Substrat permeabel ist und mindestens eine erste Fasergruppe und mindestens eine zweite Fasergruppe, deren Hydrophilie sich von der der Fasern der ersten Gruppe unterscheidet, umfasst und sich die erste und zweite Fasergruppe in derselben Schicht des Substrats befinden und derart angeordnet sind, dass eine erste Seite des Substrats auf der Außenseite des Substrats eine höhere Hydrophilie als die Hydrophilie einer zweiten Seite des Substrats aufweist, die der ersten Seite gegenüberliegt, wobei der Artikel mindestens einen hohlen oder konkaven Abschnitt umfasst und der hohle oder konkave Abschnitt dazu bestimmt ist:
iii) von einer zu behandelnden Fläche aufgenommen zu werden;
iv) ganz oder teilweise von einer Hand aufgenommen zu werden, um den Artikel mit einer zu behandelnden Fläche in Kontakt zu bringen,
wobei das Substrat aus einer Schicht besteht.

19. Kosmetikartikel nach Anspruch 18, **dadurch gekennzeichnet, dass** er in Form eines Handschuhs, eines Fäustlings, eines Däumlings, eines Füßlings, einer Haube, einer Abdeckung oder einer Abdeckhaube konfiguriert ist.

20. Kosmetikartikel nach Anspruch 18 oder 19, **dadurch gekennzeichnet, dass** der hohle oder konkave Abschnitt dazu bestimmt ist, ganz oder teilweise von einer Hand aufgenommen zu werden, um den Artikel mit einer zu behandelnden Fläche in Kontakt zu bringen, wobei die erste Seite auf der Außenseite des hohlen oder konkaven Abschnitts ist.

21. Kosmetikartikel nach Anspruch 18 oder 19, **dadurch gekennzeichnet, dass** der hohle oder konkave Abschnitt dazu bestimmt ist, eine zu behandelnde Fläche aufzunehmen, und wobei die erste Seite auf der Innenseite des hohlen oder konkaven Abschnitts ist.

## Claims

1. A disposable cosmetic article comprising a substrate impregnated with an aqueous liquid cosmetic composition, said substrate being permeable and including at least one first group of fibers and at least one second group of fibers, the hydrophilicity of which is different from that of the fibers of the first group, said first and second groups of fibers being positioned inside a same layer of the substrate and being laid out so that a first face of the substrate has a hydrophilicity substantially greater than the hydrophilicity of a second face of the substrate, opposite to the first, said first face being external to the substrate, the substrate being a monolayer substrate.

2. The cosmetic article according to claim 1, **characterized in that** the first face of the substrate has a salting index under static pressure (IRSPS1), the second face of the substrate having a salting index under static pressure (IRSPS2), the IRSPS1/IRSPS2 ratio being greater than or equal to 1.5.

3. The cosmetic article according to claim 2, **characterized in that** the IRSPS1/IRSPS2 ratio ranges from 1.5 to 15, and preferably from 2.5 to 10, and still preferably from 2.5 to 7.

4. The cosmetic article according to any of claims 1 to 3, **characterized in that** the substrate is obtained by spunlacing of a first web of said fibers of the first group and of a second web of said fibers of the second group.

5. The cosmetic article according to any of claims 1 to 4, **characterized in that** the substrate is impregnated with said cosmetic composition according to a level ranging from 100% to 1000%, preferably 150% and 800% and still preferably 150% and 400% by weight of composition per weight of non-impregnated substrate.

6. The cosmetic article according to any of the preceding claims, **characterized in that** the liquid cosmetic composition contains between 10% and 99.9% of water, and preferably between 30% and 90% of water.

7. The cosmetic article according to any of the preceding claims, **characterized in that** the liquid cosmetic composition comprises a fatty phase.

8. The cosmetic article according to the preceding claim, **characterized in that** the fatty phase represents from 0.5 to 80% by weight, and preferably from 1 to 50% by weight based on the total weight of the cosmetic composition.

9. The cosmetic article according to claim 7 or 8, **characterized in that** the fatty phase is selected from among hydrocarbon oils of animal origin, hydrocarbon oils of plant origin, synthetic esters and ethers, notably of fatty acids, linear or branched hydrocarbons of mineral or synthetic origin, fatty alcohols having from 8 to 26 carbon atoms, alkoxylated and notably ethoxylated fatty alcohols, fluorinated partly hydrocarbon and/or silicone oils, silicone oils, and mixtures thereof.

10. The cosmetic article according to any of the preceding claims, **characterized in that** the cosmetic composition further contains one or several compounds selected from among organic solvents, solubilizers, hydrophilic or lipophilic thickeners and gelling agents, softeners, anti-oxidants, opacifiers, stabilizers, foaming agents, fillers, chelating agents, perfumes, filters, essential oils, coloring materials, pigments, hydrophilic or lipophilic actives optionally encapsulating one or several actives.

11. The cosmetic article according to any of the preceding claims, **characterized in that** the cosmetic composition contains, in addition to water, one or several solvents selected from among lower alcohols including from 1 to 6 carbon atoms, such as ethanol; polyols such as glycerol; glycols such as butylene glycol, isoprene glycol, hexylene glycol, propylene glycol, polyethylene glycols such as PEG-8; sorbitol, sugars such as glucose, fructose, maltose, lactose, sucrose; and mixtures therof.

12. The cosmetic article according to any of claims 1 to 11, **characterized in that** the cosmetic composition has a viscosity of less than 1,500 mPa.s and preferably les than 1,000 mPa.s.

13. The cosmetic article according to any of the preceding claims, **characterized in that** the fibers of the first group are selected from cotton, cellulose or viscose fibers.

14. The cosmetic article according to any of the preceding claims, **characterized in that** the fibers of the second group are selected from polypropylene, polyester, polyamide or polyethylene fibers.

15. The cosmetic article according to any of the preceding claims, **characterized in that** one of said faces of the support, notably the second, is made exfoliating, notably by hot calendering.

16. The cosmetic article according to any of the preceding claims, **characterized in that** it is configured as a wipe, a pad, a glove, a mitt, a doigtier, a slipper, a cap, a headdress or a mask with the shape of the whole or part of the face.

17. The cosmetic article according to any of the preceding claims, **characterized in that** the cosmetic composition is a capillary notably dye composition or for cleaning hair, or a care composition, a self-tanning composition, a composition for removing make-up, a composition with a peeling effect, or a coloration composition.

18. A disposable cosmetic article comprising at least one substrate impregnated with a liquid cosmetic composition, said substrate being permeable and including at least one first group of fibers and at least one second group of fibers, the hydrophilicity of which is different from that of the fibers of the first group, said first and second groups of fibers being positioned inside a same layer of the substrate and being laid out so that a first face of the substrate, external to the latter, has a hydrophilicity greater than the hydrophilicity of a second face of the substrate, opposite to the first, said article including at least one recessed or concave portion, said recessed or concave portion being intended:
iii) either for receiving a surface to be treated;
iv) or for receiving all or part of a hand in order to bring the article to engagement with a surface to be treated,
the substrate being a monolayer substrate.

19. The cosmetic article according to claim 18, **characterized in that** it is configured as a glove, a mitt, a doigtier, a slipper, a cap, a headdress or a charlotte.

20. The cosmetic article according to claim 18 or 19, **characterized in that** the recessed or concave portion is intended to receive all or part of a hand in order to bring the article to engagement with a surface to be treated, the first face being outside the recessed or concave portion.

21. The cosmetic article according to claim 18 or 19, **characterized in that** the recessed or concave portion is intended to receive a surface to be treated, the first face being inside the recessed or concave portion.
